Europäisches Patentamt

European Patent Office

Office européen des brevets

(11) Numéro de publication: **0 311 866 B1**

## FASCICULE DE BREVET EUROPEEN

(45) Date de publication de fascicule du brevet: **04.03.92**  (51) Int. Cl.⁵: **A61K  35/78**

(21) Numéro de dépôt: **88116343.0**

(22) Date de dépôt: **03.10.88**

(54) Traitement des anomalies des lipoprotéines liées au métabolisme du cholesterol.

(30) Priorité: **16.10.87 CH 4069/87**

(43) Date de publication de la demande:
**19.04.89 Bulletin  89/16**

(45) Mention de la délivrance du brevet:
**04.03.92 Bulletin  92/10**

(84) Etats contractants désignés:
**AT BE DE ES FR GB GR IT LU NL SE**

(56) Documents cités:
**EP-A- 0 092 085**
**GB-A- 2 028 341**

**CHEMICAL ABSTRACTS, vol. 106, no. 19, 11 mai 1987, page 578, résumé no. 155260, Columbus, Ohio, US; H. TRAITLER et al.: "Fatty acid patterns in organ lipids in response to dietary black currant seed oil rich in gamma-linolenic acid", & PROG. LIPID RES. 1986, 25, 255-61**

**PATENT ABSTRACTS OF JAPAN, vol. 10, no. 303 (C-378)[2359], 16 octobre 1986, page 145 C 378; & JP-A-61 118 318 (POLA CHEM IND INC) 05-06-1986**

**RESSOURCES MEDICINALES DE LA FLORE**

**FRANçAISE,TOME II, 1961, pages 707-708, Vigot Frères éditeurs, Paris, FR; G. GARMIER et al.**

**Copie complete de "D.V. Horrobin et Coll. dans Lipids, vol. 18, No. 8, page 558-561".**

(73) Titulaire: **SOCIETE DES PRODUITS NESTLE S.A.**
**Service des Brevets Case postale 353**
**CH-1800 Vevey(CH)**

(72) Inventeur: **Berger, Manfred**
**Doeblingerhauptstrasse 58**
**A-1190 Vienna(AT)**
Inventeur: **Spielmann, Danièle**
**51, Avenue de Cour**
**CH-1007 Lausanne(CH)**
Inventeur: **Traitler, Helmut**
**Ch. Entre Deux Villes 5**
**CH-1802 Corseaux(CH)**

**Description**

L'invention concerne l'utilisation d'un lipide du cassis dans le traitement des anomalies des lipoprotéines liées au métabolisme du cholestérol.

Les lipides circulent dans le sang sous forme de lipoprotéines solubles en milieu aqueux constituées d'un noyau lipidique de cholestérol estérifié par des acides gras et de triglycérides entouré par une couche de protéines, de phospholipides et de cholestérol libre. L'agencement de ces divers composants caractérise le type de lipoprotéine. De nombreuses études épidémiologiques mettent en évidence le rôle prépondérant joué par les lipoprotéines dans le développement de l'athérosclérose.

En simplifiant, on admet que certaines lipoprotéines, dites de basse densité (LDL) constituent une fraction à risque cardio-vasculaire car il y a une correlation positive entre leur présence à un taux élevé dans la circulation sanguine et l'athérosclérose. Par contre, les lipoprotéines de haute densité (HDL) constituent un facteur anti-risque cardio-vasculaire car on observe une correlation négative entre leur taux et la maladie. Il y a donc un "bon cholestérol", transporté par les HDL qui drainent le cholestérol de la paroi artérielle pour le ramener au foie où il est catabolisé. Au contraire, les LDL déposent le cholestérol sur la paroi artérielle.

On sait que les huiles riches en acides gras polyinsaturés ont pour effet de baisser le taux de cholestérol total (CT) plasmatique. Cependant, dans les études effectuées, la baisse du CT est due soit à une baisse concommittante du cholestérol des LDL (CLDL) et du cholestérol des HDL (CHDL), soit à une baisse du CLDL sans modification du CHDL. D'après D.V. Horrobin et Coll. dans Lipids, vol. 18, No 8, p.558-561, ce dernier cas serait celui de l'huile d'onagre.

On sait par ailleurs de JP-A-61 118318 qu'un mélange lipidique synergique d'un alcool triterpénique cyclique spécifique synthétique et d'une huile hautement insaturée serait susceptible de réduire les taux de cholestérol libre et de cholestérol des LDL et d'augmenter le taux de cholestérol des HDL dans le plasma sanguin.

Nous avons trouvé de manière surprenante que la seule administration d'huile de pépins de cassis conduisait à faire baisser le CLDL tout en augmentant de manière significative le CHDL.

L'invention concerne donc l'utilisation d'un lipide du cassis pour fabriquer une composition pour le traitement des anomalies des lipoprotéines liées au métabolisme du cholestérol.

Sous le terme "anomalies des lipoprotéines liées au métabolisme du cholestérol", on entend:
- l'hypercholestérolémie essentielle (type IIa) caractérisée par un taux normal de triglycérides (TG), une augmentation du CT correspondant à une augmentation du CLDL et à une diminution ou un taux normal du CHDL,
- l'hyperlipidémie mixte (type IIB) caractérisée par une augmentation ou un taux normal des TG, une augmentation ou un taux normal du CT, correspondant à une augmentation du CLDL et à une diminution du CHDL,
- la dys-bêta-lipoprotéinémie (type III) caractérisée par une augmentation des TG et du CT, cette dernière étant nettement moins fréquente que les deux autres.
- l'anomalie correspondant à un taux bas de HDL.

Par lipide du cassis, on entend selon l'invention:
- l'huile de pépins de cassis (Ribes nigrum), obtenue par extraction à partir de résidus de cassis et reffinage, par exemple comme indiqué dans le brevet européen No 92.085 ou la demande de brevet européen No 137.862,
- un mélange d'acides gras provenant de l'hydrolyse ou du fractionnement de l'huile de pépins de cassis, obtenus par exemple selon la demande de brevet européen No 178.442 ou selon la demande de brevet européen No 271.747.
- un sel pharmaceutiquement acceptable des acides gras précédents,
- une huile obtenue par réestérification d'un tel mélange d'acides gras avec le glycérol,
- un mélange des lipides précédents.

Le lipide du cassis peut être avantageusement protégé de l'oxydation par un antioxydant liposoluble, par exemple le palmitate d'ascorbyle, les tocophérols ou un mélange de tels antioxydants.

Les compositions diététiques peuvent se présenter sous forme d'émulsions, par exemple des sauces, mayonnaises ou margarines.

Les compositions pharmaceutiques peuvent se présenter sous différentes formes adaptées au mode d'administration, par exemple par voie orale, entérale, rectale ou parentérale. On peut par exemple préparer des capsules, gélules, suppositoires ou sirops. Dans le cas d'une administration entérale ou parentérale, les compositions se présentent sous forme de solutions ou émulsions stabilisées physiquement et chimiquement, apyrogènes et stériles.

La dose administrée dépend du type et de la gravité de l'anomalie à traiter. Elle peut être de 1 à 25 g de lipide du cassis et de préférence de 2 à 5 g d'huile de cassis par jour en prise unique ou de préférence en 2 à 3 prises séparées.

L'exemple ci-après illustre l'invention. Dans celui-ci, les parties et pourcentages sont pondéraux sauf indication contraire.

Exemple

Conditions expérimentales

On a traité des patients ayant un taux initial de CT supérieur à 300 mg/dl pendant 12 semaines avec une dose moyenne quotidienne de 6 capsules de gélatine contenant soit 450 mg d'huile de pépins de cassis (HPC) et 200 ppm (partie par million) de palmitate d'ascorbyle, soit 450 mg d'huile de pépins de raisin (HPR) et 200 ppm de palmitate d'ascorbyle.

HPC est constituée de triglycérides des acides gras suivants, en poids

| | |
|---|---|
| acide linoléique, $C_{18:2,n-6}$ | 45% |
| acide gamma-linolénique, $C_{18:3,n-6}$ dont 38% en position béta | 17% |
| acide alpha-linolénique, $C_{18:3,n-3}$ dont 17% en position béta | 13% |
| acide stéaridonique, $C_{18:4,n-3}$ dont 32% en position béta | 3,5% |

L'acide gamma-linolénique représente le produit de l'action de la delta-6-désaturase sur l'acide linoléique.

L'acide stéaridonique constitue le produit de l'action de la delta-6-désaturase sur l'acide alpha-linolénique.

HPR contient, en poids environ 70% d'acide linoléique, 1-2% d'acide alpha-linolénique, mais pas d'acide gamma-linolénique ni d'acide stéaridonique.

Tous les patients ont obtenu une feuille d'information contenant des recommandations diététiques.

Le groupe recevant l'HPC consistait en 5 patients comprenant 3 femmes et 2 hommes d'âge moyen 61 ans (de 36 à 76). Le groupe recevant l'HPR était constitué de 7 patients, 4 femmes et 3 hommes d'âge moyen 55 ans (de 48 à 62). Dans les deux groupes les CT initiaux étaient similaires. Dans les deux groupes la plupart des malades étaient de type IIb, défini précédemment.

Des prises de sang ont eu lieu à intervalles de 0, 4, 8 et 12 semaines pour l'analyse des paramètres.

Résultats

Le tableau 1 ci-après donne les valeurs des moyennes des paramètres indiqués ($\overline{X}$) et les écarts type (ET) pour chaque visite ainsi que la probabilité du test statistique (test T) pour les deux groupes. Le test T donne une réponse à la question : y a-t-il une différence entre les moyennes des groupes étudiés et cela pour chaque visite ? Sont soulignés les cas significatifs (probabilité $\leq$ 0,05)

Le CT (mg/dl) est dosé enzymatiquement à partir du sérum par colorimétrie en utilisant les enzymes cholestérolestérase, cholestéroloxydase et le révélateur 4-amino-phénazine.

Le CHDL (mg/dl) est dosé de la même manière que le CT sur la fraction contenant les HDL recueillie après ultracentrifugation du sérum à 12.000 t/min.

Le CLDL (mg/dl) est déduit de la valeur du CHDL d'après Friedewald et Fredrickson.

EP 0 311 866 B1

Tableau 1

| GROUPES | | n | CT | | | | CHDL | | | | CLDL | | | |
|---|---|---|---|---|---|---|---|---|---|---|---|---|---|---|
| | | | 0 | 4 | 8 | 12 | 0 | 4 | 8 | 12 | 0 | 4 | 8 | 12 |
| HPC | $\overline{X}$ | 5 | 309,20 | 293,40 | 279,20 | 261,00 | 42,80 | 50,20 | 56,40 | 61,40 | 270,20 | 260,00 | 234,20 | 206,60 |
| | ET | 5 | 4,49 | 3,94 | 6,27 | 9,57 | 1,91 | 2,04 | 1,92 | 1,43 | 13,73 | 12,30 | 14,21 | 17,82 |
| HPR | $\overline{X}$ | 7 | 304,43 | 295,00 | 298,43 | 293,71 | 40,86 | 39,71 | 42,71 | 39,57 | 276,57 | 273,00 | 267,14 | 271,28 |
| | ET | 7 | 4,63 | 7,12 | 6,27 | 9,57 | 1,91 | 2,04 | 1,92 | 1,43 | 13,73 | 12,29 | 14,21 | 17,82 |
| Probabilité | | | 0,492 | 0,865 | 0,079 | 0,033 | 0,526 | 0,130 | 0,029 | 0,001 | 0,790 | 0,593 | 0,191 | 0,021 |

Le tableau 2 ci-après concerne les probabilités des tests T appariés entre les visites $V_4$, $V_8$, $V_{12}$ et $V_0$. Dans ces tests statistiques, on cherche, au niveau de chaque groupe séparément, les différences entre la visite $V_0$ et les visites successives $V_4$, $V_8$ et $V_{12}$, autrement dit l'évolution du phénomène en fonction du temps. Ici encore les cas significatifs sont soulignés (probabilité $\leq 0,05$).

Tableau 2

| GROUPE HPC | | CT | | | CHDL | | | CLDL | | |
|---|---|---|---|---|---|---|---|---|---|---|
| Paramètres | | | | | | | | | | |
| Entre visites | | $V_4-V_0$ | $V_8-V_0$ | $V_{12}-V_0$ | $V_4-V_0$ | $V_8-V_0$ | $V_{12}-V_0$ | $V_4-V_0$ | $V_8-V_0$ | $V_{12}-V_0$ |
| Probabilité | n=5 | 0,003 | 0,040 | 0,010 | 0,233 | 0,025 | 0,010 | 0,066 | 0,016 | 0,002 |
| GROUPE HPR | | | | | | | | | | |
| Probabilité | n=7 | 0,045 | 0,219 | 0,143 | 0,030 | 0,408 | 0,233 | 0,303 | 0,028 | 0,393 |

5

Dans le tableau 3 ci-après on étudie le rapport

$$\frac{CLDL}{CHDL}$$

appelé "risque standard d'athérogénicité" ainsi que les probabilités du test T. Le tableau 4 ci-après donne les probabilités des tests T appariés. Sont soulignés les cas significatifs.

Tableau 3

| Groupes | | CLDL/CHDL | | | | |
|---------|---|---|---|---|---|---|
| | | n | 0 | 4 | 8 | 12 |
| HPC | $\overline{X}$ | 5 | 6,34 | 5,63 | 4,24 | 3,48 |
| | ET | 5 | 0,44 | 0,95 | 0,34 | 0,38 |
| HPR | $\overline{X}$ | 7 | 6,86 | 6,97 | 6,35 | 6,93 |
| | ET | 7 | 0,45 | 0,42 | 0,49 | 0,56 |
| Probabilité | | | 0,445 | 0,184 | 0,010 | 0,010 |

Tableau 4

| Groupe HPC | | | | |
|------------|---|---|---|---|
| Entre visites | | $V_4$-$V_0$ | $V_8$-$V_0$ | $V_{12}$-$V_0$ |
| Probabilité | n = 5 | 0,259 | <0,001 | <0,001 |
| Groupe HPR | | | | |
| Probabilité | n = 7 | 0,298 | 0,155 | 0,740 |

Conclusions

Dans le groupe HPC, la moyenne du CHDL a augmenté nettement (+43,5%), les moyennes du CLDL et du CT ont diminué (-23,5% et -15,6% respectivement) et la moyenne du facteur risque standard d'athérogénicité a diminué de 6,34 à 3,48 au cours du traitement.

Par comparaison, les valeurs correspondantes du groupe HPR ont été :
CHDL (-3,2%), CLDL (-1,9%), CT (-3,5%)

La moyenne du facteur risque standard d'athérogénicité n'a pratiquement pas changé (de 6,86 à 6,93).

On voit donc que l'administration de HPC conduit à une chute du CT due uniquement à une baisse du CLDL sans participation du CHDL qui au contraire augmente de manière substantielle. Le risque standard d'athérogénicité diminue donc fortement.

Par contre, l'administration de HPR n'apporte aucune amélioration de l'état pathologique des patients.

**Revendications**

1.  Utilisation d'un lipide du cassis pour fabriquer une composition diététique destinée au traitement des anomalies des lipoprotéines liées au métabolisme du cholestérol.

2.  Utilisation d'un lipide du cassis pour fabriquer une composition pharmaceutique destinée au traitement des anomalies des lipoprotéines liées au métabolisme du cholestérol.

3.  Utilisation selon la revendication 1, sous une forme adaptée à l'administration par voie orale, entérale ou parentérale.

4. Utilisation selon la revendication 2, sous une forme adaptée à l'administration par voie orale, rectale, entérale ou parentérale.

5. Utilisation selon la revendication 1 ou 2, sous une forme fournissant une dose quotidienne de 1 à 25 g de lipide du cassis.

6. Utilisation selon la revendication 5, sous une forme fournissant une dose quotidienne de 2 à 5 g d'huile de cassis.

**Claims**

1. The use of a lipid of the blackcurrant for the preparation of a dietetic composition for the treatment of lipoprotein disorders associated with cholesterol metabolism.

2. The use of a lipid of the blackcurrant for the preparation of a pharmaceutical composition for the treatment of lipoprotein disorders associated with cholesterol metabolism.

3. The use claimed in claim 1 in a form suitable for oral, enteral or parenteral administration.

4. The use claimed in claim 2 in a form suitable for oral, rectal, enteral or parenteral administration.

5. The use claimed in claim 1 or 2 in a form providing a daily dose of 1 to 25 g blackcurrant lipid.

6. The use claimed in claim 5 in a form providing a daily dose of 2 to 5 g blackcurrant oil.

**Patentansprüche**

1. Verwendung eines Lipides der Schwarzen Johannesbeere für die Erzeugung einer diätetischen Zusammensetzung, welche für die Behandlung von Lipoproteinanomalien, die mit dem Cholesterinstoffwechsel verbunden sind, bestimmt ist.

2. Verwendung eines Lipides der Schwarzen Johannisbeere für die Erzeugung einer pharmazeutischen Zusammensetzung, welche für die Behandlung von Lipoproteinanomalien, die mit dem Cholesterinstoffwechsel verbunden sind, bestimmt ist.

3. Verwendung nach Anspruch 1, in einer Form, welche für eine Verabreichung auf oralem, enteralem oder parenteralem Wege angepaßt ist.

4. Verwendung nach Anspruch 2, in einer Form, welche für eine Verabreichung auf oralem, rektalem, enteralem oder parenteralem Wege angepaßt ist.

5. Verwendung nach Anspruch 1 oder 2, in einer Form, welche eine tägliche Dosis von 1 bis 25 g des Lipides der Schwarzen Johannisbeere zur Verfügung stellt.

6. Verwendung nach Anspruch 5, in einer Form, welche eine tägliche Dosis von 2 bis 5 g des Öles der Schwarzen Johannisbeere zur Verfügung stellt.